# EUROPEAN PATENT SPECIFICATION

(11) **EP 2 631 644 B1**
(45) Date of publication and mention of the grant of the patent: **10.12.2014**
(21) Application number: 12198810.9
(22) Date of filing: 21.12.2012
(51) Int. Cl.: G01N 33/46, G01N 21/898, H04N 19/60, G06T 7/00

(54) **Providing a strength related factor of wood material**
Bereitstellung eines festigkeitsbezogenen Faktors von Holzwerkstoff
Fourniture d'un facteur apparenté de résistance d'un matériau dérivé du bois

(30) Priority: 21.12.2011 FI 20116300
(43) Date of publication of application: 28.08.2013
(73) Proprietor: UPM-Kymmene Wood Oy, 15140 Lahti (FI)
(72) Inventor: Koponen, Simo, 15140 Lahti (FI); Luukkainen, Juha-Pekka, 58200 Kerimäki (FI); Korpijaakko, Saija, 15300 Lahti (FI); Kiuru, Jani, 54100 Joutseno (FI)
(74) Representative: Papula Oy

(56) References cited:
- WO-A1-2005/010628
- "Strength of Wood, Fundamental Relationships", , 1998, XP055071077, Retrieved from the Internet: URL:http://ifbholz.ethz.ch/natureofwood/pc /st/st5.html [retrieved on 2013-07-12]

## Description

### FIELD OF THE INVENTION:

The invention relates generally to wood veneers and machine vision. In particular, the invention relates to providing a strength related factor of wood veneer based on a compressed digital image size indication.

### BACKGROUND OF THE INVENTION:

Document US 2003/0042180 discloses increasing strength and/or reducing strength variations in multilayer wood, plywood, and the like sandwich material by measuring wood veneers for dry substance density with high-frequency electromagnetic resonance and additionally analyzing the homogeneity and/or grain structure of wood veneers from a wood veneer surface in the way of its darkness.

### SUMMARY OF THE INVENTION:

A first aspect of the present invention is a method of providing a strength related factor of wood material in which at least one digital image of at least a portion of wood material is obtained. A data size indication of at least a region of the obtained and compressed at least one digital image is generated. A strength related factor of the at least the portion of the wood material is determined based on the generated data size indication, the strength related factor correlating inversely with the data size indication.

A second aspect of the present invention is computer program that comprises code adapted to cause the execution of the method of the first aspect when executed on a data-processing system. The computer program may be stored on a computer readable medium.

A third aspect of the present invention is an apparatus for providing a strength related factor of wood material, which comprises a digital image obtaining unit configured to obtain at least one digital image of at least a portion of wood material. The apparatus further comprises a data size indication generation unit configured to generate a data size indication of at least a region of the obtained and compressed at least one digital image. The apparatus further comprises a strength related factor determination unit configured to determine a strength related factor of the at least the portion of the wood material based on the generated data size indication, said strength related factor correlating inversely with said data size indication.

A fourth aspect of the present invention is an apparatus for providing a strength related factor of wood material, which comprises a digital image obtaining means for obtaining at least one digital image of at least a portion of wood material. The apparatus further comprises a data size indication generating means for generating a data size indication of at least a region of the obtained and compressed at least one digital image. The apparatus further comprises a strength related factor determining means for determining a strength related factor of the at least the portion of the wood material based on the generated data size indication, said strength related factor correlating inversely with said data size indication.

A fifth aspect of the present invention is a system which comprises a production line that is configured to process a mat of wood material. The system further comprises at least two of the apparatuses of the fourth aspect which are arranged at different locations on the production line.

In an embodiment of the invention, the generation of the data size indication comprises adjusting brightness of the at least one region.

In an embodiment of the invention, the generation of the data size indication further comprises converting the at least one region into a duotone form.

In an embodiment of the invention, in the generation of the data size indication, the compression of the at least the region of the obtained at least one digital image comprises JPEG compression of quality of substantially 30%-50%.

In an embodiment of the invention, the adjustment of the brightness comprises increasing the brightness by substantially 20%.

In an embodiment of the invention, the data size indication comprises one of data size and data size estimate of the at least the region of the obtained and compressed at least one digital image.

In an embodiment of the invention, the generation of the data size indication comprises: performing a Fourier transformation on the at least the region of the obtained at least one digital image resulting in a matrix having values corresponding to pixels of the at least the region of the obtained at least one digital image. In an embodiment of the invention, the Fourier transformation comprises a discrete cosine transformation.

In an embodiment of the invention, the generation of the data size indication further comprises weighing the matrix values based on frequency; and converting the weighted matrix values to integers.

In an embodiment of the invention, the data size indication comprises the inverse of the amount of substantial zeros among the matrix values.

In an embodiment of the invention, the determination of the strength related factor further comprises utilizing information about density of the at least the portion of the wood material.

In an embodiment of the invention, the information about the density is obtained via radiating the at least the portion of the wood material with beta radiation.

In an embodiment of the invention, the wood material comprises a mat of the wood material being processed on a production line, and the obtaining of the digital image comprises obtaining at least two digital images from different locations on the production line. In this embodiment of the invention, the determined strength related factors based on the multiple digital images obtained from the different locations may be utilized in controlling a drying process of the mat of the wood material.

In an embodiment of the invention, the strength related factor comprises at least one of: strength, elastic modulus, and surface hardness.

In an embodiment of the invention, the wood material comprises wood veneer.

It is to be understood that the aspects and embodiments of the invention described above may be used in any combination with each other. Several of the aspects and embodiments may be combined together to form a further embodiment of the invention. A method, an apparatus, a system or a computer program which is an aspect of the invention may comprise at least one of the embodiments of the invention described above.

The invention allows providing strength related factor of wood material that is more accurate than prior art solutions, yet at the same time more cost-effective and faster to produce and use.

### BRIEF DESCRIPTION OF THE DRAWINGS:

The accompanying drawings, which are included to provide a further understanding of the invention and constitute a part of this specification, illustrate embodiments of the invention and together with the description help to explain the principles of the invention. In the drawings:
**Fig. 1a** is a flow diagram illustrating a method according to an embodiment of the invention;
**Fig. 1b** is a flow diagram illustrating a method according to another embodiment of the invention;
**Fig. 1c** is a flow diagram illustrating a method according to yet another embodiment of the invention;
**Fig. 2** is a block diagram illustrating an apparatus according to an embodiment of the invention;
**Fig. 3a** is a diagram illustrating a production line related to a net dryer according to an embodiment of the invention; and
**Fig. 3b** is a diagram illustrating a production line related to a roller dryer according to an embodiment of the invention.

### DETAILED DESCRIPTION OF THE PREFERRED EMBODIMENTS:

Reference will now be made in detail to the embodiments of the invention, examples of which are illustrated in the accompanying drawings.

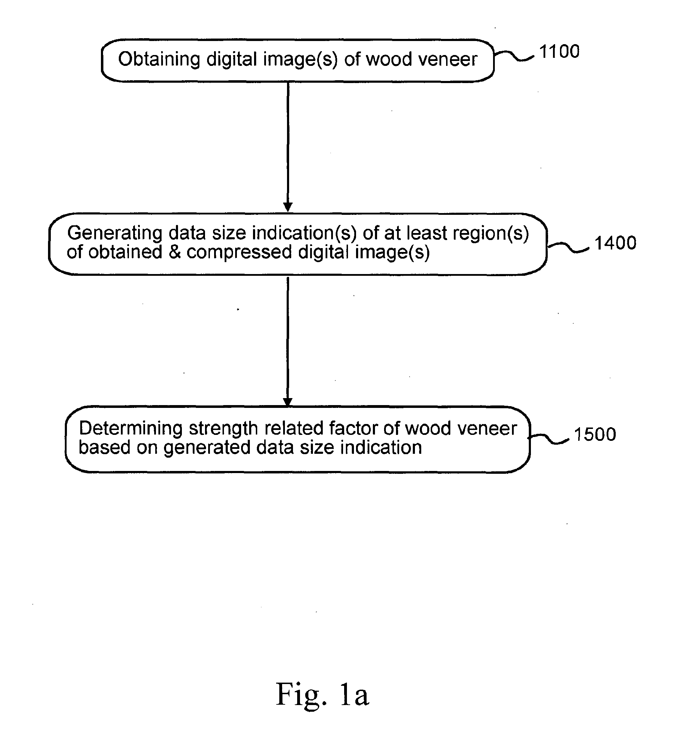

Figure 1a is a flow diagram illustrating a method of providing a strength related factor of wood material according to an embodiment of the invention.

Herein, the term "wood material" includes a solid piece of wood, a wood product and a wood veneer. Mechanical properties of wood material vary depending e.g. on the wood species, density, annual ring width, slope of grain, defects and how it is manufactured and cut from the tree.

Herein, the term "wood product" is used to refer to a piece of sawn timber, veneer sheet or plywood panel. Mechanical properties of wood product vary depending on the wood material used and on the manufacture process.

Herein, the term "wood veneer" is used to refer to a thin sheet of wood material normally having thickness less than 4 mm, and peeled from the log.

Plywood consists of three or more layers of veneer, each glued with its grain at right angle to adjacent layers. In some cases partially orientated veneer lay-up may be glued then having parallel grain direction in some of the adjacent veneer layers. Laminated veneer lumber (LVL) is a strongly orientated plywood type wooden product, having normally the grain direction of all the veneers in parallel to each other.

In the following description, wood veneer is used as an example. However, the invention is not limited to wood veneer.

At step 1100, at least one digital image of at least a portion of wood veneer is obtained. The at least one digital image may be captured with e.g. a digital camera, such as a digital line camera, still camera, infrared camera, or a video camera. The image may be composed of e.g. visible light frequencies, infrared (IR) radiation, near-infrared (NIR) radiation, or a combination of any of the above. The camera(s) may be arranged such that it(them) can be utilized in capturing images of wood veneer. Such arrangements may include e.g. lighting, focusing and the like, which are known to those of average skill in the art, and thus are not described in detail here. In an embodiment, the wood veneer comprises long veneer mats (e.g. approximately 15-30 meters in length) peeled from a log. The wood veneer or veneer mats may be arranged on a conveyor belt or a production line, wherein the veneers or veneer mats are continuously moving while the digital camera remains fixed in its position.

The captured digital images may be uncompressed images, images compressed in a lossless manner, or images compressed in a lossy manner. Furthermore, the captured digital images may be color images or black and white images.

Size of the captured digital images may be e.g. M x N pixels. In an embodiment, N may be e.g. in the range of 512-4096. In an embodiment, M ≥ 1. In an embodiment, there may be e.g. 1-3 color channels in the captured digital images.

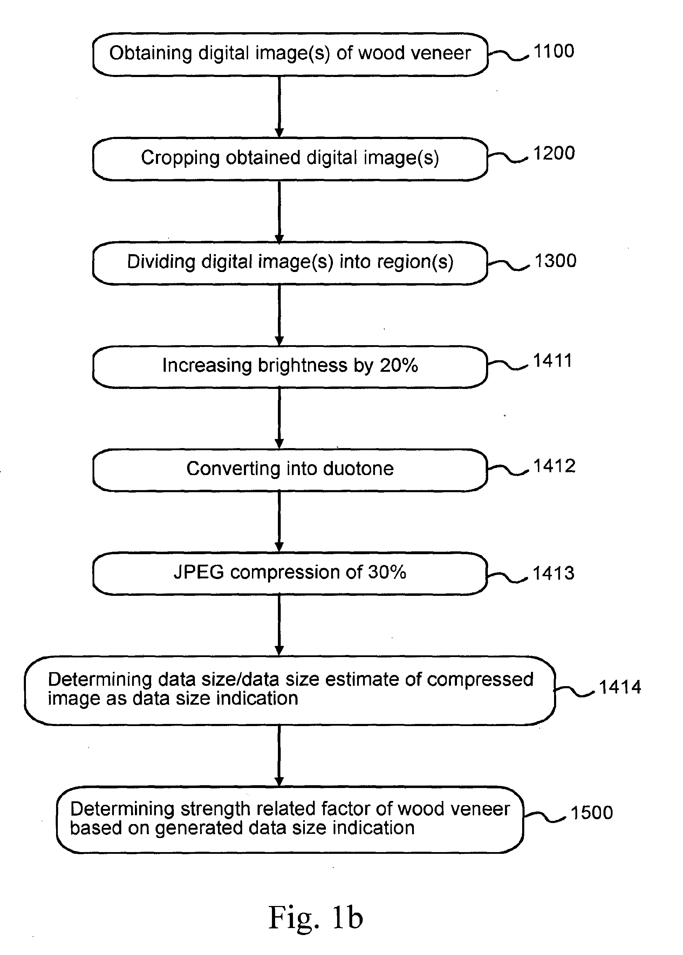

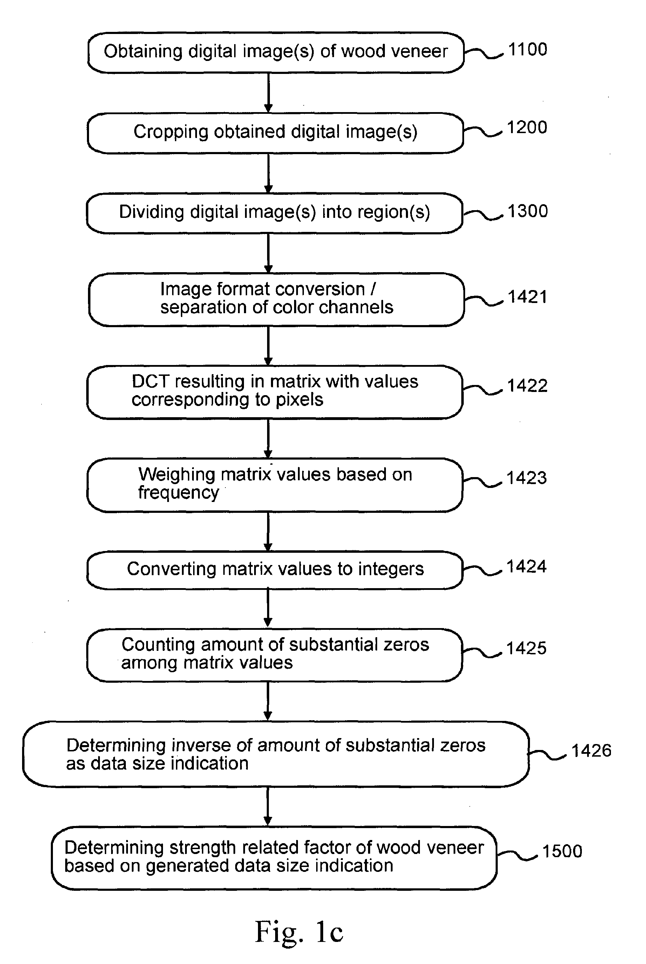

At step 1400, a data size indication of at least a region of the obtained and compressed at least one digital image is generated. Various more detailed descriptions of examples of how to implement step 1400 are provided below in connection with Figures 1b and 1c.

At step 1500, a strength related factor of the at least the portion of the wood veneer is determined based on the generated data size indication. The strength related factor correlates inversely with the data size indication.

In other words, the invention is based on the observation made by the present inventors that the data size of a compressed digital image captured from wood veneer correlates inversely with the strength properties of the wood veneer. This is due to the fact that the data size of a compressed digital image depends mainly on brightness and color variations within the image. When the image is of wood veneer, various defects such as knots, holes, decay, slope of grain, annular ring width, early wood proportion, etc., which affect strength properties negatively, will cause these brightness variations (dark and light areas) within the image. Therefore, an image captured from a portion of veneer with a large amount of defects decreasing the strength properties of the respective portion will have a larger data size or file size than an image captured from a portion of veneer with no defects or only a small amount of defects. In other words, the stronger the portion of veneer, the smaller the data size of the compressed image captured from it. Different types of defects can be separated using different conversion tables. Thus, the data size of the compressed image, or an estimate of the data size, or an indication of the data size may be used as tool facilitating grading of veneers to different strength classes in an accurate, cost-effective and efficient manner.

The strength related factor may comprise at least one of: strength, elastic modulus, and surface hardness.

The embodiments described below in connection with Figure 1b and 1c provide further enhancements to generating the data size indication to maximize its accuracy in indicating the strength properties, and to maximize the efficiency of its generation process as well as the efficiency of its utilization.

Figure 1b is a flow diagram illustrating a method of providing a strength related factor of wood veneer according to another embodiment of the invention.

In the embodiment of Figure 1b, step 1400 of Figure 1a comprises sub-steps 1411-1414. Steps 1100 and 1500 are similar to Figure 1a, and therefore are not described again here.

At step 1200, the obtained at least one digital image is cropped. The cropping may performed by selecting an area of pixels inside of which there is only wood veneer.

At step 1300, the obtained at least one digital image is divided into regions. In an embodiment, the image is divided into four regions. Additionally/alternatively, the regions may be e.g. 8 x 8 pixels.

At step 1411, brightness of at least one region is adjusted. In an embodiment, the brightness is increased by substantially 20%. Naturally, other brightness level changes may be used.

At step 1412, the at least one region is converted into a duotone form. Again, this conversion further highlights defects. As is known in the art, duotone is a halftone reproduction of an image using the superimposition of one contrasting color halftone over another color halftone.

At step 1413, the at least one region is JPEG compressed, e.g. by substantially 30%-50%. Naturally, other compression methods and compression percentages may be used.

At step 1414, the data size or an estimate of the data size of the thus produced compressed image or image region is taken as the data size indication, and it will be used as such in step 1500.

Figure 1c is a flow diagram illustrating a method of providing a strength related factor of wood veneer according to yet another embodiment of the invention.

In the embodiment of Figure 1c, step 1400 of Figure 1a comprises sub-steps 1421-1426. Steps 1100 and 1500 are similar to Figure 1a, and therefore are not described again here. Also, steps 1200 and 1300 are similar to Figure 1b, and therefore are not described again here.

At step 1421, image format is converted and/or color channels are separated. For example, pixel value levels may be converted, such as from levels 0 to 255 to levels -128 to +128, in case of color depth being 8 bits. Furthermore, a transformation from RGB space to YUV space may be performed.

At step 1422, a Fourier transformation, such as a discrete cosine transformation (DCT) is performed on the at least the region of the obtained at least one digital image resulting in a matrix (or a vector) having values corresponding to pixels of the at least the region of the obtained at least one digital image. As described above, the region may be e.g. 8 x 8 pixels, but regions of other sizes may also be used.

As is known in the art, Fourier transformation (FT) is a mathematical operation that decomposes a signal into its constituent frequencies. Discrete cosine transform (DCT) is a Fourier-related transformation similar to discrete Fourier transformation (DFT). In digital image compression algorithms, DCT is typically used due to its efficiency. Both of these transformations (DCT and DFT) can be one- or two-dimensional operations.

At step 1423, the matrix values are weighed based on spatial frequency or orientation. The matrix resulting from the DCT typically contains a lot of matrix values close to zero. Since the human eye cannot distinguish rapid variations in color values of neighboring pixels (i.e. high frequencies in a cosine transformation), high spatial frequencies may be equalized / removed e.g. by utilizing a quantization table to weigh the matrix values based on spatial frequencies of terms of cosine series, thereby increasing the amount of matrix values of zero. The lowest spatial frequency may be in the term D[0,0] of the matrix which corresponds to the average brightness or color tones (gray scale Y, colors U and V) of the image region being analyzed.

At step 1424, the weighted matrix values are converted to integers. Alternatively, they may be separated to low and high values.

At step 1425, the amount of substantial zeros among the matrix values is counted. In an embodiment, zeros may be counted for each color channel and image region. Also, the number of zeros and D[0,0] terms for each color channel and image region may be stored.

At step 1426, the inverse of the amount of substantial zeros among the matrix values is taken as the data size indication, and it will be used as such in step 1500. Compression ratio is proportional to the amount of zero values in the matrix with good accuracy. Therefore, in this embodiment, the inverse of the amount of zero values is utilized as the data size indication.

As discussed above, for the digital image or for the region of the digital image a discrete Fourier transform or discrete cosine transform is calculated. The result matrixes can be divided using a quantization table which has standard values used in image compression or experimentally tailored values to suit for the determination of the strength related factor. Then real numbers in the elements of the obtained new matrix are rounded to integers.

Alternatively, the elements of the DFT or DCT result matrix can be compared to an experimentally determined reference table. The absolute values of the result matrix lower than the reference matrix values have no effect to the strength and can thus be converted to zero values.

In both methods, the number of zero values in the image or in the region of the image compared to the number of elements in the image or in the region of interest indicates the compression ratio of the whole image or the compression ratio of the region of the image. The strength indicator for the studied wood material is the image size indicator multiplied by the experimentally determined constant. E.g. regression analysis may be used to determine that constant.

In digital image compression, the compressed image size depends on the size of the original image, on the target quality of the final decompressed image, and on the efficiency of the compression algorithm. Herein, compressed image size indication may comprise the number of zeros obtained after e.g. the discrete cosine transformation and dividing the matrix with the quantification table and rounding result matrix to integer values. In the invention, the compression ratio indicator may comprise the obtained number of zero values divided with the number of all elements in the matrix. The compression ratio indicator is strongly correlated to the actual compressed image size having equal target image quality. Thus the compression ratio based on the actual compressed image size or compression ratio indicator can be used to predict strength properties. To minimize calculation work and to speed up the analysis, in fixed measuring systems having always the same size of the original image, the compressed image size or compressed image size indicator can also be used directly to predict strength properties.

The determination of the strength related factor may further comprise utilizing information about density of the at least the portion of the wood material. The information about the density may be obtained e.g. via radiating the at least the portion of the wood material with e.g. beta radiation. For example, the wood veneer may comprise a mat of the wood veneer being processed on a production line, and the obtaining of the digital image may comprise obtaining at least two digital images, each from a different location on the production line. In this embodiment, the determined strength related factors based on the multiple digital images obtained from the different locations may be utilized e.g. in controlling a drying process of the mat of the wood material.

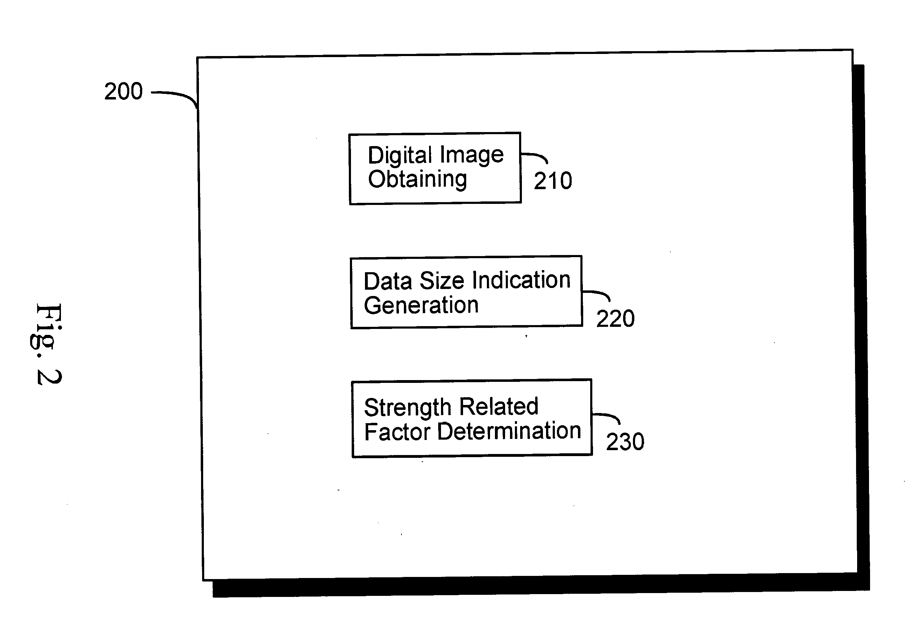

Figure 2 is a block diagram illustrating an apparatus 200 for providing a strength related factor of wood veneer according to an embodiment of the invention. The apparatus 200 may comprise e.g. a personal computer, a workstation, a computer server, or the like, which may be connected to the digital camera that is used to capture the digital image(s) of the at least the portion of the wood veneer. Alternatively, the apparatus 200 or at least portions of it may be e.g. integrated in the digital camera itself.

The apparatus 200 comprises a digital image obtaining unit 210 that is configured to obtain at least one digital image of at least a portion of wood veneer. The apparatus 200 further comprises a data size indication generation unit 220 that is configured to generate a data size indication of at least a region of the obtained and compressed at least one digital image. The apparatus 200 further comprises a strength related factor determination unit 230 that is configured to determine a strength related factor of the at least the portion of the wood veneer based on the generated data size indication, the strength related factor correlating inversely with the data size indication.

In an embodiment of the invention, the data size indication generation unit 220 may further be configured to perform the generation of the data size indication by adjusting brightness of the at least one region. The data size indication generation unit 220 may further be configured to perform the generation of the data size indication by converting the at least one region into a duotone form. The data size indication generation unit 220 may further be configured to perform the compression of the at least the region of the obtained at least one digital image by JPEG compression of quality of substantially 30%-50%. The data size indication generation unit 220 may further be configured to perform the adjusting of the brightness by increasing the brightness by substantially 20%. In this embodiment, the data size indication may comprise one of data size and data size estimate of the at least the region of the obtained and compressed at least one digital image.

In another embodiment of the invention, the data size indication generation unit 220 may further be configured to perform the generation of the data size indication by performing a Fourier transformation, such as a discrete cosine transformation on the at least the region of the obtained at least one digital image resulting in a matrix having values corresponding to pixels of the at least the region of the obtained at least one digital image. The data size indication generation unit 220 may further be configured to perform the generation of the data size indication by weighing the matrix values based on frequency; and converting the weighted matrix values to integers. In this embodiment, the data size indication may comprise the inverse of the amount of substantial zeros among the matrix values.

The strength related factor determination unit 230 may further be configured to determine the strength related factor by utilizing information about density of the at least the portion of the wood material. The strength related factor determination unit 230 may further be configured to obtain the information about the density based on beta radiation radiated on the at least the portion of the wood material.

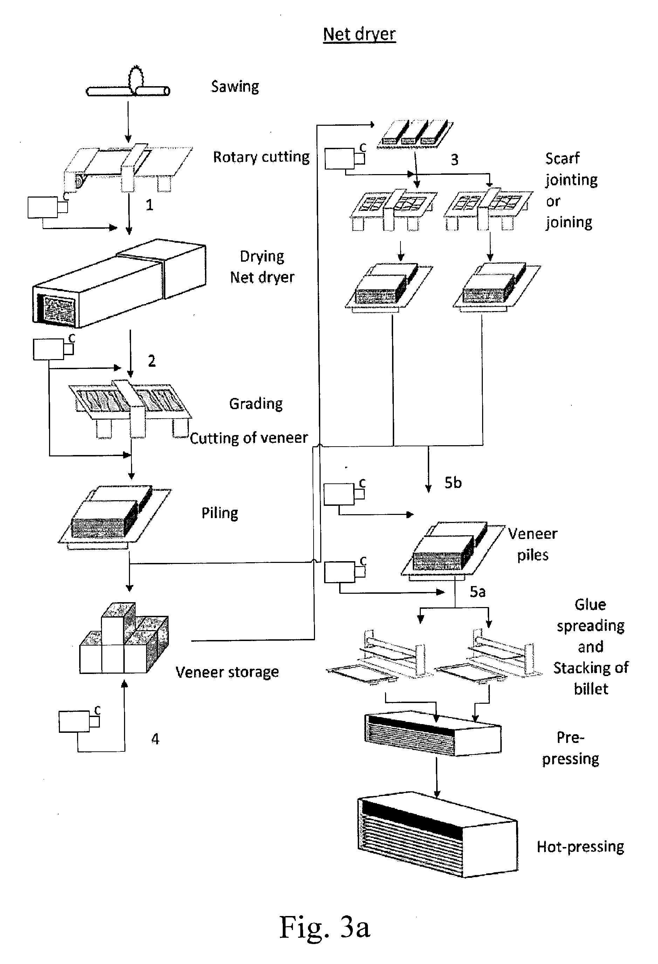

Next, utilization of the present invention is described with reference to Figures 3a-3b which illustrate production lines related to a net dryer and a roller dryer, respectively. Numerals 1-5b are used to indicate examples of locations to which to deploy apparatuses 200 of the invention, thereby allowing their respective digital image obtaining units 210 to obtain digital images from multiple different locations on the production line. Then, the determined strength related factors based on the multiple digital images obtained from the different locations may be utilized e.g. in controlling a drying process of the mat of the wood material.

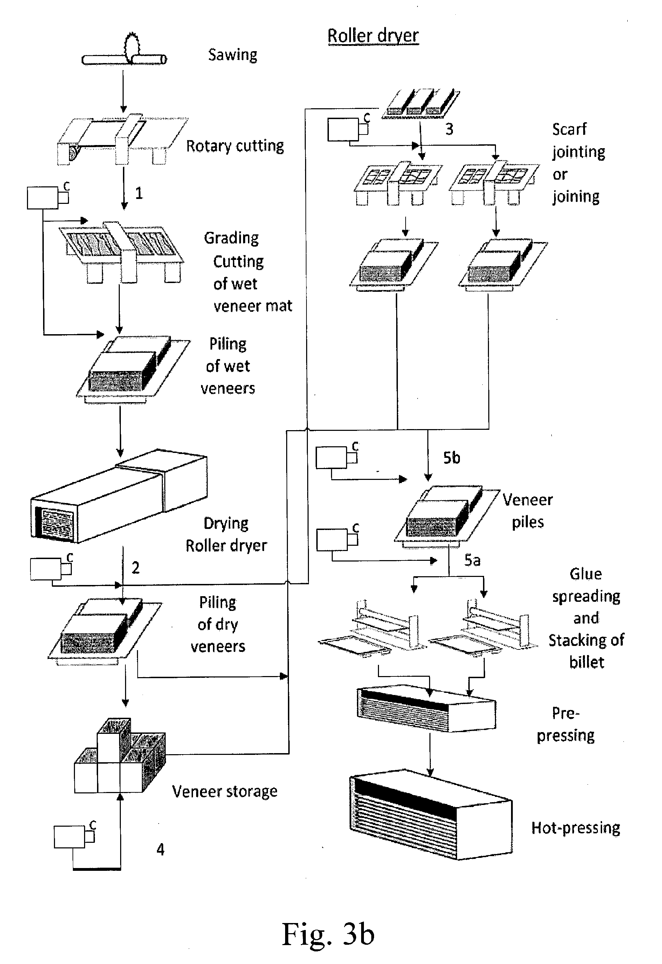

Location 1: in the case of roller drying (Figure 3b), a wet veneer mat (typically length is 1.3m-2,5m and width is 10m-25m) is cut to sheets (typically width is 1.3m-2,5m) before drying. In this case, the cutting and strength grading of veneers may be done utilizing visual strength indication obtained with a camera located after peeling and before cutting. Strength indication correlates with the initial green moisture content of veneer, with the drying speed and with the variation of the final moisture content in the dried veneer. Thus, strength indication can also be used as a moisture indication to grade veneers and then dry different veneer grades using an optimized drying formula. Normally, the optimum moisture content of dried veneers is 6%, but commonly most of the veneers are over dried to the average moisture content of about 3% to avoid too wet veneers. The advantages of the invention are savings in drying energy, shorter drying time, more constant moisture content of veneers after drying and improved handling properties of veneers in later manufacture process stages and reduction of waste and disturbances. Also, the better drying result improves the end use properties of plywood such as glue line strength, bending strength and dimensional stability.

In the case of a net dryer (Figure 3a), visual strength indication may also be used for measurements of veneer mat to control conveyer speed and/or humidity and temperature inside the drier. The measurement of green density of veneer simultaneously with the strength indication measurements improves the accuracy of strength indication and the efficiency of process control.

Location 2: in the case of the net dryer (Figure 3a), the visual strength indication may be measured from the dried veneer mat after the drier but before cutting. The density or weight per unit area of veneer and moisture content of veneer can also be measured in this process point to improve the grading result or to be used as a feedback for the drier control. In the case of drier control, an infrared camera may be used to measure strength indication. After the cutting, veneers may be stacked based on strength indication to different strength classes or sorted at conveyers for the later processes in plywood manufacture.

Location 3: typically the length of the veneer sheet is 1.3-2.6m. In scarf jointing of veneers, larger veneer sheets in the longitudinal direction of veneer are glued to allow producing large size plywood panels. Typically scarf jointing units have two or more gluing machines, each having their own stacking station. When the camera is located before the gluing units, the veneers can be driven to different gluing units based on the strength indication and scarf jointed veneer sheets can be graded to separate piles. In butt joining, veneer are jointed in the width direction and then stacked to piles. In this case strength indication can be measured before joining or after joining but before stacking of veneers.

Location 4: the measurements of strength indication and grading of the veneers can be done at the veneer storage with a separate independent grading unit. The grade of the existing veneer pile can be verified, or one veneer pile may be divided to a plurality of veneer piles based on the strength indication.

Locations 5a-5b: the measurements of the strength indication can be done before the glue spreader and/or before the assembly of veneer to plywood billet. When there are several glue spreading/assembly units, the veneer sheet can be directed to the unit where it is the most optimal to have that strength grade and that quality veneer. The assembly of plywood billet may be synchronized between different gluing/assembly units such a manner that fluent material flow and optimized veneer usage in plywood lay-up can be realized. If there is only one gluing/assembly unit in use, the veneer sheet having a strength indication not fulfilling a predefined limit can be directed to an additional veneer pile to wait for later use.

Furthermore, in the point of process and end use properties' optimization, strength indications measured from at least two different measuring points (1, 2, 3, 4 or 5) may be combined.

One or more interface mechanisms can be used with the exemplary embodiments, including, for example, Internet access, telecommunications in any suitable form (e.g., voice, modem, and the like), wireless communications media, and the like. For example, employed communications networks or links can include one or more wireless communications networks, cellular communications networks, 3G communications networks, Public Switched Telephone Network (PSTNs), Packet Data Networks (PDNs), the Internet, intranets, a combination thereof, and the like.

It is to be understood that the exemplary embodiments are for exemplary purposes, as many variations of the specific hardware used to implement the exemplary embodiments are possible, as will be appreciated by those skilled in the hardware and/or software art(s). For example, the functionality of one or more of the components of the exemplary embodiments can be implemented via one or more hardware and/or software devices.

The exemplary embodiments can store information relating to various processes described herein. This information can be stored in one or more memories, such as a hard disk, optical disk, magnetooptical disk, RAM, and the like. One or more databases can store the information used to implement the exemplary embodiments of the present inventions. The databases can be organized using data structures (e.g., records, tables, arrays, fields, graphs, trees, lists, and the like) included in one or more memories or storage devices listed herein. The processes described with respect to the exemplary embodiments can include appropriate data structures for storing data collected and/or generated by the processes of the devices and subsystems of the exemplary embodiments in one or more databases.

The exemplary embodiments can be conveniently implemented using one or more general purpose processors, microprocessors, digital signal processors, micro-controllers, and the like, programmed according to the teachings of the exemplary embodiments of the present inventions, as will be appreciated by those skilled in the computer and/or software art(s). Appropriate software can be readily prepared by programmers of ordinary skill based on the teachings of the exemplary embodiments, as will be appreciated by those skilled in the software art. In addition, the exemplary embodiments can be implemented by the preparation of application-specific integrated circuits or by interconnecting an appropriate network of conventional component circuits, as will be appreciated by those skilled in the electrical art(s). Thus, the exemplary embodiments are not limited to any specific combination of hardware and/or software.

Stored on any one or on a combination of computer readable media, the exemplary embodiments of the present inventions can include software for controlling the components of the exemplary embodiments, for driving the components of the exemplary embodiments, for enabling the components of the exemplary embodiments to interact with a human user, and the like. Such software can include, but is not limited to, device drivers, firmware, operating systems, development tools, applications software, and the like. Such computer readable media further can include the computer program product of an embodiment of the present inventions for performing all or a portion (if processing is distributed) of the processing performed in implementing the inventions. Computer code devices of the exemplary embodiments of the present inventions can include any suitable interpretable or executable code mechanism, including but not limited to scripts, interpretable programs, dynamic link libraries (DLLs), Java classes and applets, complete executable programs, Common Object Request Broker Architecture (CORBA) objects, and the like. Moreover, parts of the processing of the exemplary embodiments of the present inventions can be distributed for better performance, reliability, cost, and the like.

As stated above, the components of the exemplary embodiments can include computer readable medium or memories for holding instructions programmed according to the teachings of the present inventions and for holding data structures, tables, records, and/or other data described herein. Computer readable medium can include any suitable medium that participates in providing instructions to a processor for execution. Such a medium can take many forms, including but not limited to, non-volatile media, volatile media, transmission media, and the like. Non-volatile media can include, for example, optical or magnetic disks, magneto-optical disks, and the like. Volatile media can include dynamic memories, and the like. Transmission media can include coaxial cables, copper wire, fiber optics, and the like. Transmission media also can take the form of acoustic, optical, electromagnetic waves, and the like, such as those generated during radio frequency (RF) communications, infrared (IR) data communications, and the like. Common forms of computerreadable media can include, for example, a floppy disk, a flexible disk, hard disk, magnetic tape, any other suitable magnetic medium, a CD-ROM, CD±R, CD±RW, DVD, DVD-RAM, DVD±RW, DVD±R, HD DVD, HD DVD-R, HD DVD-RW, HD DVD-RAM, Blu-ray Disc, any other suitable optical medium, punch cards, paper tape, optical mark sheets, any other suitable physical medium with patterns of holes or other optically recognizable indicia, a RAM, a PROM, an EPROM, a FLASH-EPROM, any other suitable memory chip or cartridge, a carrier wave or any other suitable medium from which a computer can read.

While the present inventions have been described in connection with a number of exemplary embodiments, and implementations, the present inventions are not so limited, but rather cover various modifications, and equivalent arrangements, which fall within the purview of prospective claims.

## Claims

1. A method of providing a strength related factor of wood material, comprising:
obtaining (1100) at least one digital image of at least a portion of wood material;
**characterized in** further comprising:
generating (1400) a data size indication of at least a region of the obtained and compressed at least one digital image; and
determining (1500) a strength related factor of the at least the portion of the wood material based on the generated data size indication, said strength related factor correlating inversely with said data size indication.

2. The method according to claim 1, wherein the step of generating (1400) the data size indication comprises at least one of:
adjusting (1411) brightness of the at least one region; and
converting (1412) the at least one region into a duotone form.

3. The method according to claim 2, wherein the data size indication comprises (1414) one of data size and data size estimate of the at least the region of the obtained and compressed at least one digital image.

4. The method according to claim 1, wherein the step of generating (1400) the data size indication comprises:
performing (1422) a Fourier transformation on the at least the region of the obtained at least one digital image resulting in a matrix having values corresponding to pixels of the at least the region of the obtained at least one digital image.

5. The method according to claim 4, wherein the data size indication comprises (1426) the inverse of the amount of substantial zeros among the matrix values.

6. The method according to any of claims 1-5, wherein the step of determining (1500) the strength related factor further comprises utilizing information about density of the at least the portion of the wood material; and the method further comprising obtaining the information about the density via radiating the at least the portion of the wood material with beta radiation.

7. The method according to any of claims 1-6, wherein the strength related factor comprises at least one of: strength, elastic modulus, and surface hardness.

8. A computer program comprising code adapted to cause the execution of any of claims 1-7 when executed on a data-processing system.

9. An apparatus (200) for providing a strength related factor of wood material, comprising:
a digital image obtaining unit (210) configured to obtain at least one digital image of at least a portion of wood material;
**characterized in** the apparatus (200) further comprising:
a data size indication generation unit (220) configured to generate a data size indication of at least a region of the obtained and compressed at least one digital image; and
a strength related factor determination unit (230) configured to determine a strength related factor of the at least the portion of the wood material based on the generated data size indication, said strength related factor correlating inversely with said data size indication.

10. The apparatus (200) according to claim 9, wherein the data size indication generation unit (220) is further configured to perform the generation of the data size indication by at least one of adjusting brightness of the at least one region and converting the at least one region into a duotone form.

11. The apparatus (200) according to claim 10, wherein the data size indication comprises one of data size and data size estimate of the at least the region of the obtained and compressed at least one digital image.

12. The apparatus (200) according to claim 9, wherein the data size indication generation unit (220) is further configured to perform the generation of the data size indication by performing a Fourier transformation on the at least the region of the obtained at least one digital image resulting in a matrix having values corresponding to pixels of the at least the region of the obtained at least one digital image.

13. The apparatus (200) according to claim 12, wherein the data size indication comprises the inverse of the amount of substantial zeros among the matrix values.

14. The apparatus (200) according to any of claims 9-13, wherein the strength related factor comprises at least one of: strength, elastic modulus, and surface hardness.

15. A system, comprising:
a production line (1-5b) configured to process a mat of wood material; and
at least two of the apparatuses (200) according to any of claims 9-14, arranged at different locations on the production line, with their respective digital image obtaining units (210) configured to obtain digital images from the different locations on the production line;
wherein the system is configured to utilize the determined strength related factors based on the multiple digital images obtained from the different locations in controlling a drying process of the mat of the wood material.

## Patentansprüche

1. Verfahren zum Bereitstellen eines festigkeitsbezogenen Faktors eines Holzwerkstoffes, umfassend:
Erhalten (1100) wenigstens eines digitalen Bildes von zumindest einem Teil eines Holzwerkstoffes;
**gekennzeichnet dadurch, dass** das Verfahren ferner umfasst:
Erzeugen (1400) einer Datengrößeangabe von zumindest einem Bereich des erhaltenen und komprimierten, wenigstens einen digitalen Bildes; und
Bestimmen (1500) eines festigkeitsbezogenen Faktors von dem zumindest dem Teil des Holzwerkstoffs, der auf der erzeugten Datengrößeangabe basiert, wobei der festigkeitsbezogene Faktor mit der Datengrößeangabe invers korreliert.

2. Verfahren nach Anspruch 1, wobei der Schritt des Erzeugens (1400) der Datengrößeangabe wenigstens eines aus Folgendem umfasst:
Anpassen (1411) der Helligkeit von dem zumindest einen Bereich; und
Konvertieren (1412) von dem zumindest einen Bereich in eine zweifarbige Form.

3. Verfahren nach Anspruch 2, wobei die Datengrößeangabe entweder eine Datengröße oder eine Datengrößenabschätzung von dem zumindest dem Bereich, des erhaltenen und komprimierten, wenigstens einen digitalen Bildes umfasst (1414).

4. Verfahren nach Anspruch 1, wobei der Schritt des Erzeugens (1400) der Datengrößeangabe umfasst:
Ausführen (1422) einer Fourier Transformation auf den zumindest den Bereich des erhaltenen wenigstens einen digitalen Bildes, das in einer Matrix mit Werten resultiert, die den Pixeln von dem zumindest dem Bereich des erhaltenen, wenigstens einen digitalen Bildes entsprechen.

5. Verfahren nach Anspruch 4, wobei die Datengrößeangabe die Inverse der Menge von im Wesentlichen Nullen unter den Matrixwerten umfasst (1426).

6. Verfahren nach einem der Ansprüche 1 - 5, wobei der Schritt des Bestimmens (1500) des festigkeitsbezogenen Faktors ferner umfasst, eine Information über eine Dichte von dem zumindest dem Teil des Holzwerkstoffs zu verwenden; und wobei das Verfahren ferner umfasst, die Formation über die Dichte mittels Bestrahlen von dem zumindest dem Teil des Holzwerkstoffs mit Betastrahlung zu erhalten.

7. Verfahren nach einem der Ansprüche 1 - 6, wobei der festigkeitsbezogene Faktor wenigstens eines aus Folgendem umfasst: eine Festigkeit, ein Elastizitätsmodul, und eine Oberflächenhärte.

8. Computerprogramm umfassend einen Code, der angepasst ist, die Ausführung einer der Ansprüche 1 - 7 herbeizuführen, wenn dieses in einem Datenverarbeitungssystem ausgeführt wird.

9. Vorrichtung (200) zum Bereitstellen eines festigkeitsbezogenen Faktors eines Holzwerkstoffes, umfassend:
eine Einheit zum Erhalten digitaler Bilder (210), die konfiguriert ist, um wenigstens ein digitales Bild von zumindest einem Teil des Holzwerkstoffs zu erhalten;
**gekennzeichnet dadurch, dass** die Vorrichtung (200) ferner umfasst:
eine Einheit zum Erzeugen einer Datengrößeangabe (220), die konfiguriert ist, um eine Datengrößeangabe von zumindest einem Teil des erhaltenen und komprimierten, wenigstens einen digitalen Bildes zu erzeugen; und
eine Einheit zum Bestimmen eines festigkeitsbezogenen Faktors (230), die konfiguriert ist, um einen festigkeitsbezogenen Faktor von dem zumindest dem Teil des Holzwerkstoffes, der auf der erzeugten Datengrößeangabe basiert zu bestimmen, wobei der festigkeitsbezogene Faktor mit der Datengrößeangabe invers korreliert.

10. Vorrichtung (200) nach Anspruch 9, wobei die Einheit zum Erzeugen der Datengrößeangabe (220) ferner konfiguriert ist, um die Erzeugung der Datengrößeangabe mittels wenigstens einer der folgenden Möglichkeiten, wie der Helligkeitsanpassung des zumindest einen Bereichs und dem Konvertieren des zumindest einen Bereichs in eine zweifarbige Form durchzuführen.

11. Vorrichtung (200) nach Anspruch 10, wobei die Datengrößeangabe entweder von einer Datengröße oder einer Datengrößeabschätzung von dem zumindest dem Bereich des erhaltenen und komprimierten, wenigstens einen digitalen Bildes umfasst.

12. Vorrichtung (200) nach Anspruch 9, wobei die Einheit zur Erzeugung einer Datengrößeangabe (220) ferner konfiguriert ist, um die Erzeugung einer Datengrößeangabe durch das Ausführen einer Fourier Transformation auf den zumindest den Bereich des erhaltenen wenigstens einen digitalen Bildes, das in einer Matrix mit Werten resultiert, die den Pixeln von dem zumindest dem Bereich des erhaltenen wenigstens einen digitalen Bildes entsprechen, durchzuführen.

13. Vorrichtung (200) nach Anspruch 12, wobei die Datengrößeangabe die Inverse der Menge von im Wesentlichen Nullen unter den Matrixwerten umfasst.

14. Vorrichtung (200) nach einem der Ansprüche 9 - 13, wobei der festigkeitsbezogene Faktor wenigstens eines von Folgendem umfasst: eine Festigkeit, ein Elastizitätsmodul, und eine Oberflächenhärte.

15. System, umfassend:
eine Fertigungslinie (1-5b), die konfiguriert ist, um eine Unterlage aus Holzwerkstoff zu verarbeiten; und
wenigstens zwei der Vorrichtungen (200) nach einem der Ansprüche 9 - 14, die an verschiedenen Orten auf der Fertigungslinie angeordnet sind, wobei sie mit ihren entsprechenden Einheiten zum Erhalten eines digitalen Bildes (210) so konfiguriert sind, um digitale Bilder von den verschiedenen Orten auf der Fertigungslinie zu erhalten;
wobei das System konfiguriert ist, um die bestimmten festigkeitsbezogenen Faktoren, die auf den mehreren digitalen Bildern von den verschiedenen Orten basieren, zu verwenden, um einen Trocknungsprozess der Unterlage aus dem Holzwerkstoff zu steuern.

## Revendications

1. Procédé de fourniture d'un facteur lié à la résistance d'un matériau dérivé du bois, comprenant l'étape consistant à :
obtenir (1100) au moins une image numérique d'au moins une portion de matériau dérivé du bois ;
**caractérisé en ce qu'**il comprend en outre les étapes consistant à :
générer (1400) une indication de taille des données d'au moins une région de ladite au moins une image numérique obtenue et compressée ; et
déterminer (1500) un facteur lié à la résistance de ladite au moins une portion du matériau dérivé du bois sur la base de l'indication de taille des données générée, ledit facteur lié à la résistance étant en corrélation inverse avec ladite indication de taille des données.

2. Procédé selon la revendication 1, dans lequel l'étape de génération (1400) de l'indication de taille des données comprend au moins l'une des actions suivantes :
le réglage (1411) de la luminosité de ladite au moins une région ; et
la conversion (1412) de ladite au moins une région sous une forme à deux tons.

3. Procédé selon la revendication 2, dans lequel l'indication de taille des données comprend (1414) soit une taille des données soit une estimation de taille des données de ladite au moins une région de ladite au moins une image numérique obtenue et compressée.

4. Procédé selon la revendication 1, dans lequel l'étape de génération (1400) de l'indication de taille des données comprend l'étape consistant à :
exécuter (1422) une transformation de Fourier sur ladite au moins une région de ladite au moins une image numérique obtenue en obtenant une matrice ayant des valeurs correspondant à des pixels de ladite au moins une région de ladite au moins une image numérique obtenue.

5. Procédé selon la revendication 4, dans lequel l'indication de taille des données comprend (1426) l'inverse de la quantité de zéros substantiels parmi les valeurs de matrice.

6. Procédé selon l'une quelconque des revendications 1 à 5, dans lequel l'étape de détermination (1500) du facteur lié à la résistance comprend en outre l'utilisation d'informations relatives à la densité de ladite au moins une portion de matériau dérivé du bois ; et le procédé comprenant en outre l'obtention des informations relatives à la densité par irradiation de ladite au moins une portion de matériau dérivé du bois avec des rayonnements béta.

7. Procédé selon l'une quelconque des revendications 1 à 6, dans lequel le facteur lié à la résistance comprend au moins un des éléments suivants : la résistance, le module d'élasticité et la dureté de surface.

8. Programme d'ordinateur comprenant un code adapté pour provoquer l'exécution de l'une quelconque des revendications 1 à 7 quand il est exécuté sur un système de traitement de données.

9. Dispositif (200) pour fournir un facteur lié à la résistance d'un matériau dérivé du bois, comprenant :
une unité d'obtention d'images numériques (210) configurée pour obtenir au moins une image numérique d'au moins une portion de matériau dérivé du bois ;
**caractérisé en ce que** le dispositif (200) comprend en outre :
une unité de génération d'indication de taille des données (220) configurée pour générer une indication de taille des données d'au moins une région de ladite au moins une image numérique obtenue et compressée ; et
une unité de détermination de facteur lié à la résistance (230) configurée pour déterminer un facteur lié à la résistance de ladite au moins une portion de matériau dérivé du bois sur la base de l'indication de taille des données générée, ledit facteur lié à la résistance étant en corrélation inverse avec ladite indication de taille des données.

10. Dispositif (200) selon la revendication 9, dans lequel l'unité de génération d'indication de taille des données (220) est en outre configurée pour effectuer la génération de l'indication de taille des données par au moins une des actions suivantes : le réglage de la luminosité de ladite au moins une région et la conversion de ladite au moins une région sous une forme à deux tons.

11. Dispositif (200) selon la revendication 10, dans lequel l'indication de taille des données comprend soit une taille des données soit une estimation de taille des données de ladite au moins une région de ladite au moins une image numérique obtenue et compressée.

12. Dispositif (200) selon la revendication 9, dans lequel l'unité de génération d'indication de taille des données (220) est configurée en outre pour effectuer la génération de l'indication de taille des données en effectuant une transformation de Fourier sur ladite au moins une région de ladite au moins une image numérique obtenue en obtenant une matrice ayant des valeurs correspondant à des pixels de ladite au moins une région de ladite au moins une image numérique obtenue.

13. Dispositif (200) selon la revendication 12, dans lequel l'indication de taille des données comprend l'inverse de la quantité de zéros substantiels parmi les valeurs de matrice.

14. Dispositif (200) selon l'une quelconque des revendications 9 à 13, dans lequel le facteur lié à la résistance comprend au moins un des éléments suivants : la résistance, le module d'élasticité et la dureté de surface.

15. Système comprenant :
une ligne de production (1-5b) configurée pour traiter un mat de matériau dérivé du bois ; et
au moins deux dispositifs (200) selon l'une quelconque des revendications 9 à 14, agencés dans des emplacements différents de la ligne de production, avec leurs unités d'obtention d'images numériques (210) respectives configurées pour obtenir des images numériques des différents emplacements sur la ligne de production ;
dans lequel le système est configuré pour utiliser les facteurs liés à la résistance déterminés sur la base des multiples images numériques obtenues à partir des différents emplacements dans la commande d'un processus de séchage du mat de matériau dérivé du bois.
